# EUROPEAN PATENT APPLICATION

(11) **EP 0 749 722 A2**
(43) Date of publication of application: **27.12.1996**
(21) Application number: 96106039.9
(22) Date of filing: 17.04.1996
(51) Int. Cl.: A61B 8/00

(54) **Handheld transthoracic rotatable ultrasound transducer**

(30) Priority: 22.06.1995 US 494576
(71) Applicant: Hewlett-Packard Company, Palo Alto, California 94304 (US)
(72) Inventor: Peszynski, Michael, Nowburyport, MA 01950 (US); Arling, Robert S., North Andover, MA 01845 (US)
(74) Representative: Schoppe, Fritz, Dipl.-Ing.

(57) **Abstract**

A handheld ultrasound transducer (10) rotates an image plane (18) about a reference axis by mechanically rotating a sensor array (50) behind a stationary acoustic window (42). An acoustic coupling (40) layer disposed between the sensor array (50) and acoustic window (42) provides an acoustical impedance match between the sensor array (50) and a patient. In a first embodiment, a handheld transthoracic ultrasound transducer has an acoustic window (42) that is axially symmetric at the surface of contact with a patient and the reference axis (15) is parallel to a longitudinal axis of a housing (70). In a second embodiment a handheld abdominal ultrasound transducer has a reference axis (15) that is perpendicular to a longitudinal axis (72) of the housing (70).

## Description

### Background And Summary Of The Invention

Physicians use handheld ultrasound transducers to view internal body structures such as organs and tissue from outside of a patient's body. When connected to an ultrasound imaging system, the ultrasound transducer may provide images of the heart useful for diagnosing heart ailments or soft tissue images of the abdomen indispensable for monitoring pregnancies. While the internal body structures are inherently three-dimensional, the ultrasound transducer is limited to producing a two-dimensional (2-d) image confined within an image plane. The physician is then challenged to construct a three-dimensional mental image of the internal body structure from a sequence of two-dimensional views. For example, different chambers and valves of the heart may be viewed by aiming an ultrasound beam through a narrow opening between the ribs and then precisely rotating the image plane. If the image plane is rotated about a reference axis to acquire a sequence of spatially correlated 2-d images, an actual three-dimensional (3-d) image of internal body structures may be obtained using 3-d mathematical reconstruction.

Prior art transducers of the type taught by U.S. patent number 5,159,931 which issued on November 3, 1992 mechanically rotate the image plane by rotating a probe tip, making it difficult for the physician to obtain a sequence of 2-d images. The relative motion between the probe tip and the patient's skin may inadvertently shift the reference axis, causing the ultrasound beam to be obstructed by the ribs or lungs, or destroying spatial correlation of the 2-d images necessary for 3-d mathematical reconstruction.

In accordance with a first illustrated preferred embodiment of the present invention a handheld transthoracic ultrasound transducer enables a physician to see a sequence of two-dimensional images of a patient's heart through a single opening in the ribs. A mechanically rotated sensor array rotates the image plane behind a stationary acoustic window. The acoustic window is axially symmetrical and stationary at the surface of contact with the patient, providing equal viewing of internal structures regardless of the rotational orientation of the probe. The ultrasound beam is electronically steered to define the image plane which is mechanically rotated, allowing a sequence of two-dimensional images to be obtained without shifting the reference axis.

In accordance with a second illustrated preferred embodiment of the present invention a handheld abdominal ultrasound transducer contains a stationary acoustic window and a mechanically rotated sensor array that are mounted perpendicular to a longitudinal axis of the handheld ultrasound transducer, useful for abdominal imaging.

### Brief Description Of The Drawings

Figure 1 shows a perspective view of a handheld transthoracic ultrasound transducer that is constructed in accordance with a first preferred embodiment of the present invention.

Figure 2 shows a cross-sectional view of the handheld transthoracic ultrasound transducer of Figure 1.

Figure 3 shows a perspective view of a handheld abdominal ultrasound transducer that is constructed in accordance with a second embodiment of the present invention.

### Detailed Description Of The Preferred Embodiment

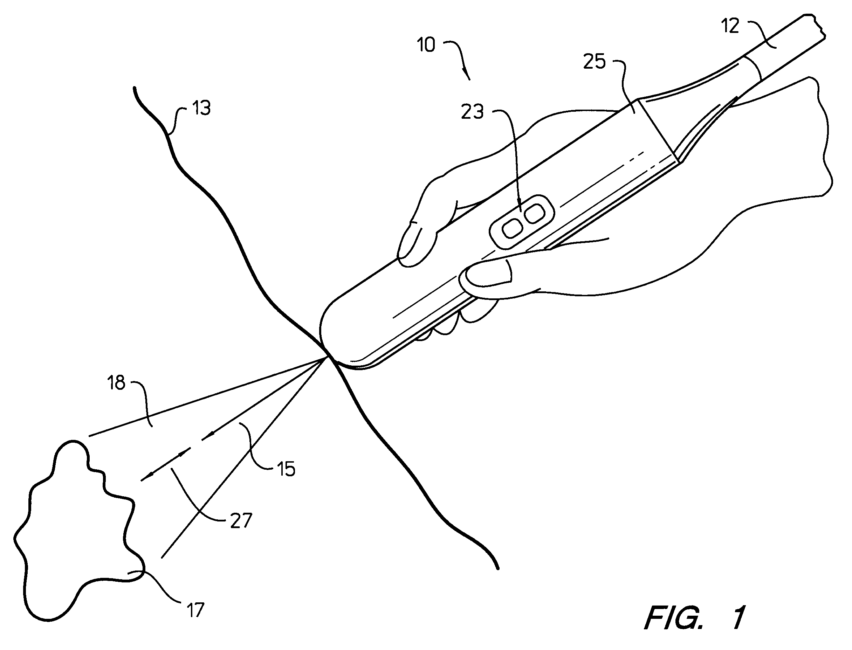

Figure 1 shows a perspective view of a handheld transthoracic ultrasound transducer 10 that is constructed in accordance with a first embodiment of the present invention. The ultrasound transducer 10 is connected to a remote ultrasound imaging system (not shown) by a transducer cable 12. A housing 25 is placed in contact with a patient's skin 13 and a reference axis 15 is coaxial with a longitudinal axis 27 (shown schematically) of the housing. The reference axis 15 is aimed at an internal body structure 17. The ultrasound transducer 10 forms an ultrasound beam defining an image plane 18 which contains the reference axis 15. A two-dimensional (2-d) image of the internal body structure 17 intercepted by the image plane 18 may be observed on the remote ultrasound imaging system.

Often a sequence of 2-d images of internal body structures is needed by the physician in order to make a diagnosis. If the image plane 18 is rotated about the reference axis 15 to a second position, a second 2-d view of the internal body structure can be obtained. Similarly, a sequence of 2-d images at predetermined rotational positions may provide the physician with enough information to construct a three-dimensional mental image of the internal body structure 17. A sequence of two-dimensional images in which each image is acquired at a known rotational position relative to the reference axis 15 provides spatial correlation needed to mathematically construct a three-dimensional (3-d) image.

The handheld transthoracic ultrasound transducer provides excellent views of the heart in 2-d cardiac imaging. Because the housing 25 is held stationary relative to the patient, the physician may easily aim the reference axis 15 at the internal body structure 17 of interest and readily control the rotational orientation of the image plane 18 by activating a switch 23 located on the housing 25. Alternatively, the rotational position of the image plane 18 can be electronically controlled through the transducer cable 12 by a pre-programmed sequence generated within the remote ultrasound imaging system or by user controls on the remote ultrasound imaging system. The handheld transthoracic ultrasound transducer 10 is axis-symmetrical relative to the reference axis 15 at the surface of contact with the patient's skin 13. This enables the physician to aim the reference axis 15 through the ribs to view the heart equally unobstructed, regardless of the rotational orientation of the housing 25.

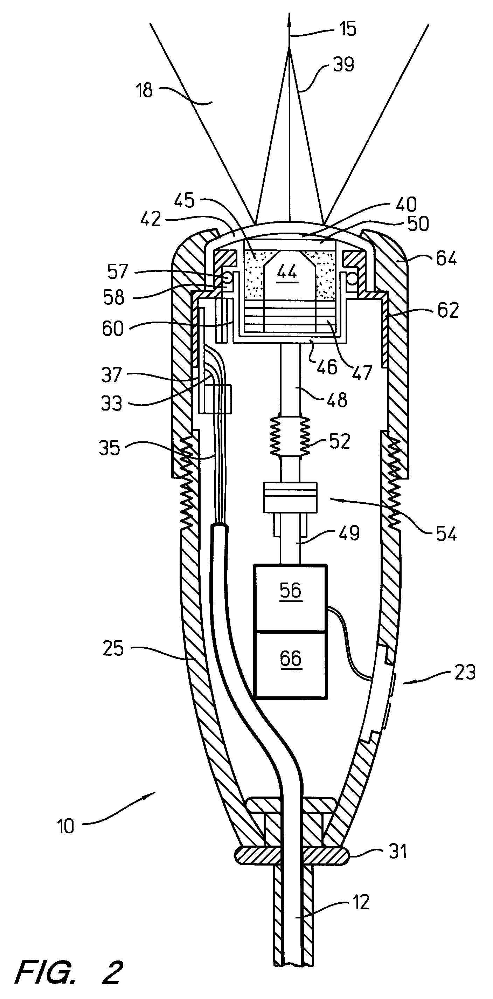

Figure 2 shows a cross-sectional view of the handheld transthoracic ultrasound transducer 10. The transducer cable 12 is connected at a distal end to a remote ultrasound imaging system (not shown) and enters the interior of the housing 25 through a strain-relief fitting 31. The proximal end of the transducer cable 12 terminates at an interconnection point 33 at which electrical conductors 35 within the transducer cable connect to a flexible circuit 37. The flexible circuit provides electrical connections to a sensor array 50.

A transmitted electrical signal (not shown) is generated within the remote ultrasound imaging system and propagates through the transducer cable 12 and flexible circuit 37 to the sensor array 50. The sensor array converts the electrical signal into an ultrasound beam 39 shown schematically. The direction of ultrasound beam may be steered back and forth within the image plane 18 by progressively delaying an electrical signal applied to separate array elements (not shown) within the sensor array 50. The image plane 18 in this embodiment is perpendicular to the sensor array 50 and contains the reference axis 15.

The ultrasound beam 39 propagates from the sensor array 50 and through an acoustic coupling fluid 40 and an acoustic window 42. The acoustic window is placed in contact with the patient's skin and the fluid 40 provides an acoustical impedance match between the sensor array 50 and the acoustic window 42 to minimize reflections of the ultrasound beam between the patient and the array sensor 50. The ultrasound beam 39 then propagates into the internal body structures where it is attenuated and reflected by internal organs and tissue. The reflected ultrasound beam is then received by the sensor array 50 and converted back into a received electrical signal (not shown) which propagates via the transducer cable 12 back to the remote ultrasound imaging system. The received electrical signal is then analyzed by the remote ultrasound imaging system to generate a 2-d image of the internal body structure within the image plane 18.

The sensor array 50 is mounted on top of an acoustic backing 45 which absorbs any backward-travelling ultrasound energy emitted by the sensor array 50. The acoustic backing 45 also provides mechanical support for the sensor array 50. A thermal conduction path for the sensor array 50 is provided by a heat sink 44 internal to the acoustic backing 45. The heat sink 44 is positioned far enough away from the sensor array 50, so that it will not cause acoustic reverberation and degrade the 2-d image quality. The acoustic backing 45 and heat sink 44 are mounted on a wave breaker 47 constructed from a composite of materials having significantly differing acoustic impedances and is designed to absorb ultrasound energy. The wave breaker 47 is also thermally conductive. The wave breaker 47, acoustic backing 45 and sensor array 50 are mounted on a turntable 46 connected to a drive shaft 48 which is rotated by an electric motor 56 located within the housing 25. A flexible coupling 52 provides rotational coupling between the electric motor 56 and the drive shaft 48. A speed reducing assembly 54 matches the speed and torque of the electric motor 56 to the load of the turntable 46. Rotational position can be measured using a position sensing encoder 66 such as a resistive potentiometer and communicated through the transducer cable 12 to the remote ultrasound imaging system. Switch 23 may be located on the housing 25 to actuate the rotation of the turntable 46 and sensor array 50 about the reference axis 15. The 2-d image plane 18 defined by the electronically steered ultrasound beam 39 may be rotated by rotating the sensor array 50.

The rotation of the sensor array 50, wave breaker 47 and turntable 46 is facilitated by a set of bearings 57 located in a channel 58 defined by a retaining wall 60 continuous with the turntable 46 and a bearing housing 62 in contact with a window mount 64. The acoustic window 42 is stationary within the window mount 64 which is attached to the housing 25. In this embodiment, the acoustic window 42 may be fabricated from polyester, Mylar or another similar thin shell material, attached to the bearing housing 62 by epoxy or similar adhesive. The acoustic window 42 acts as a stationary interface between the patient and sensor array 50. The sensor array 50 can then rotate while the acoustic window 42 does not move relative to the patient's skin. The acoustic coupling fluid 40 located between the acoustic window 42 and the sensor array 50 is a layer of flourosilicone, silicone or other similar oil held in place by the surface tension between the two. The acoustic coupling fluid 40 has low attenuation to the ultrasound beam 39 and provides rotational coupling between the rotating sensor array 50 and the stationary acoustic window 40.

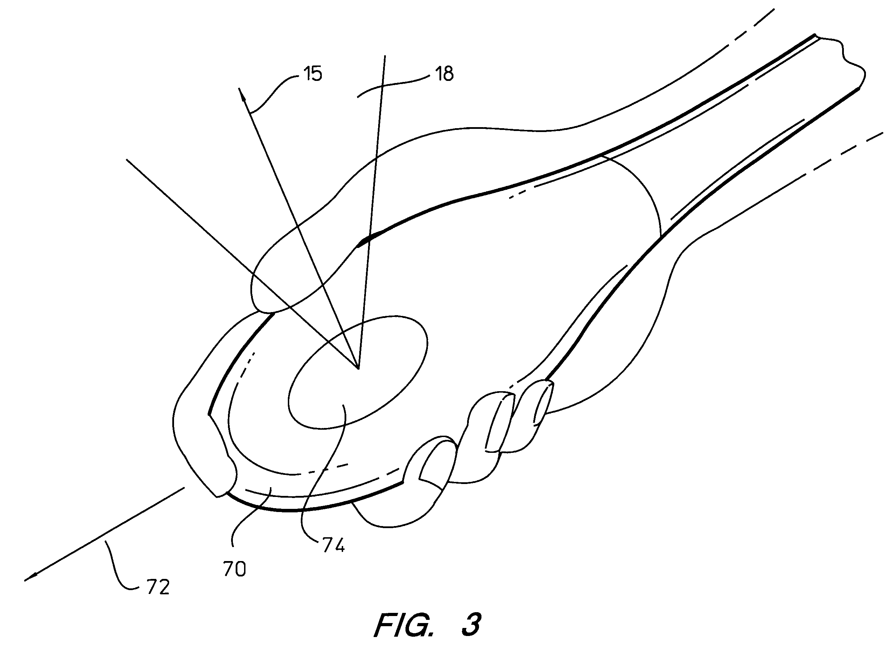

Flexible coupling 52 permits the sensor array 50, acoustic window 42 and other elements mounted on the turntable 46 to be positioned at an angle relative to the electric motor 56. Figure 3 shows a perspective view of a handheld abdominal ultrasound transducer 70 that is constructed in accordance with a second embodiment of the present invention. In this embodiment, the reference axis 15 and image plane 18 are orthogonal to a longitudinal axis 72 (shown schematically) of the abdominal ultrasound transducer 70. This abdominal ultrasound transducer 70 can be positioned with a stationary acoustic window 74 placed on the abdomen of a patient to obtain soft tissue images of the organs in the abdomen or to monitor pregnancies. Once the reference axis 15 is aimed at the internal structure of interest, the image plane 18 can be rotated about the reference axis 15 to obtain a sequence of 2-d images. Similar to the first embodiment of the present invention, the abdominal ultrasound transducer 70 has a sensor array (not shown) that electronically directs an ultrasound beam back and forth within the image plane 18 and then mechanically rotates a sensor array (not shown) behind the stationary acoustic window 74 and a acoustic coupling fluid (not shown). Because there is no relative motion between the stationary acoustic window 74 and the patient's skin, a sequence of 2-d images rotated about the reference axis 15 of the abdomen can be easily obtained.

## Claims

1. An ultrasound transducer (10) assembly for use with a remote ultrasound imaging system, comprising:
a housing (25), said housing having an aperture, therein;
a planar sensor array (50) rotatably mounted within the housing (25), having an image plane (18) perpendicular to the plane of the array for obtaining a corresponding two-dimensional cross-sectional image relative to each rotational position of the array;
a stationary acoustic window (42) within the aperture;
an acoustic coupling layer (40) positioned between the sensor array (50) and the acoustic window;
a cable (12) attached to the housing (25) for connecting the sensor array (50) with the remote ultrasound imaging system; and
a motor (56), within the housing, for rotating the sensor array (50).

2. The ultrasound transducer assembly of claim 1 wherein the image plane is defined by an electronically scanned ultrasound beam.

3. The ultrasound transducer assembly of claim 2 wherein the image plane further comprises a reference axis (15) about which the image plane (18) is rotated corresponding to the rotation of the sensor array (50) by the motor (56).

4. The ultrasound transducer assembly of claim 3 further comprising a flexible coupling (52) between the motor (56) and the sensor array (50).

5. The ultrasound transducer (10) assembly of claim 4 wherein the reference axis (15) is parallel to the longitudinal axis.

6. The ultrasound transducer (10) assembly of claim 5 wherein the acoustic window (41) is circular.

7. The ultrasound transducer (10) assembly of claim 4 wherein the reference axis (15) is perpendicular to the longitudinal axis (72).

8. The ultrasound transducer assembly of claim 4 wherein the housing (25) further comprises a switch (23) for controlling the motor (56).

9. The ultrasound transducer assembly of claim 3 wherein the sensor array (50) is rotated to a predetermined sequence of rotational positions.

10. A method for obtaining a spatially correlated sequence of two-dimensional ultrasound images of a subject, comprising the steps of;
positioning a stationary acoustic window in contact with the subject;
electronically directing an ultrasound beam to acquire a first two-dimensional image within an image plane; and
mechanically rotating the image plane about a reference axis to acquire a second two-dimensional image.

11. The method of claim 10 wherein mechanically rotating the image plane further comprises the step of rotating a sensor array acoustically coupled to the stationary acoustic window.
